(19) Europäisches Patentamt
European Patent Office
Office européen des brevets

(11) Publication number: **0 167 197 B1**

# (12) EUROPEAN PATENT SPECIFICATION

(45) Date of publication of patent specification: **18.01.89**

(21) Application number: **85200956.2**

(22) Date of filing: **18.06.85**

(51) Int. Cl.[4]: **C 07 C 127/22,** C 07 D 213/75, C 07 D 213/79, C 07 D 213/80, A 01 N 47/34, A 01 N 47/36

## (54) Benzoylurea compounds, and insecticidal and acaricidal compositions comprising same.

(30) Priority: **05.07.84 NL 8402137**

(43) Date of publication of application:
**08.01.86 Bulletin 86/02**

(45) Publication of the grant of the patent:
**18.01.89 Bulletin 89/03**

(84) Designated Contracting States:
**AT BE CH DE FR GB IT LI LU NL SE**

(56) References cited:
**EP-A-0 050 321**
**EP-A-0 069 288**
**EP-A-0 131 071**
**DE-A-2 928 410**
**DE-A-3 046 672**
**DE-A-3 133 009**
**DE-A-3 334 224**
**NL-A-7 905 155**
**US-A-4 264 605**

(73) Proprietor: **DUPHAR INTERNATIONAL RESEARCH B.V**
**C.J. van Houtenlaan 36**
**NL-1381 CP Weesp (NL)**

(72) Inventor: **Brouwer, Marius S.**
**c/o OCTROOIBUREAU ZOAN B.V. Apollolaan 151**
**NL-1077 AR Amsterdam (NL)**
Inventor: **Grosscurt, Arnoldus C.**
**c/o OCTROOIBUREAU ZOAN B.V. Apollolaan 151**
**NL-1077 AR Amsterdam (NL)**
Inventor: **van Hes, Roelof**
**c/o OCTROOIBUREAU ZOAN B.V. Apollolaan 151**
**NL-1077 AR Amsterdam (NL)**

(74) Representative: **Swaters, Pieter D. et al**
**OCTROOIBUREAU ZOAN B.V. P.O. Box 140**
**NL-1380 AC Weesp (NL)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The invention relates to new benzoylurea compounds and to a method of preparing said compounds. The invention also relates to compositions having insecticidal and acaricidal activity and comprising these compounds, and to the use of said compositions for controlling insects and/or mites.

It is already known that certain N-benzoyl-N'-phenylurea compounds have an insecticidal activity. From Netherlands Patent Application 7105350 in the name of Applicants it appears that in particular the substitution pattern of the benzoyl group has an important influence on said activity. In general, a high insecticidal activity is found in benzoylurea compounds the benzoyl group of which is substituted in the 2- or 2,6-positions, for example, with one or two halogen atoms. Substituents on the other side of the molecule, so in the N'-phenyl ring, are less essential for the insecticidal activity but can nevertheless influence said activity in such manner that the benzoylurea compound is better or worse suitable for practical applications. For example, it appears from an article by Wellinga et al. in J. Arg. Food Chem., *Vol.* 21, No. 3, 1973, *pp.* 348—354 that electrons-donating substituents at the N'-phenyl ring, for example, a methoxy group, adversely influence the insecticidal activity.

It has surprisingly been found, that N'-phenyl benzoylurea compounds, to the phenyl group of which are attached *via* an oxygen bridge, an oxycarbonyl bridge, an oxycarbonylamino bridge or an alkoxy bridge a whether or not substituted cycloalkyl or cycloalkenyl group or a bi- or polycyclic hydrocarbyl group, having an interesting insecticidal activity. Moreover, an acaricidal activity is also found in the new benzoylurea compounds according to the invention.

Chemically related benzoylurea compounds are described in Netherlands Patent Application 7905155 in the name of Applicants, e.g. N-(2-chlorobenzoyl)-N'-[4-(1-phenylcyclohexyloxy)phenyl]urea.

As will be clear from the Examples, this known compound is considerably less active than the new compounds of the present invention.

The invention relates to benzoylurea compounds of the general formula

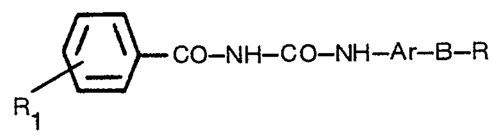

(I)

wherein

$R_1$ is a hydrogen atom or represents 1 or 2 halogen atoms;

Ar is a para-phenylene group or a para-pyridylene group, which groups may be substituted with 1 or 2 substituents selected from the group consisting of chlorine, methyl and trifluoromethyl;

B is an oxygen atom or represents a group having the formula

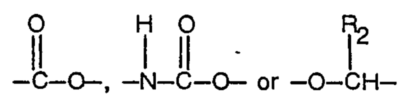

wherein $R_2$ is a hydrogen atom or an alkyl group having 1—4 carbon atoms; and

R is a cycloalkyl group or a cycloalkenyl group having 6—12 carbon atoms, which groups may be substituted with 1—3 substituents selected from the group consisting of $C_1$—$C_6$ alkyl and $C_2$—$C_6$ alkenyl, or wherein

R is a bi- or polycyclic, saturated or unsaturated, non-aromatic hydrocarbyl group, having 8—14 carbon atoms and, if desired, substituted with 1—3 substituents selected from the group consisting of $C_1$—$C_6$ alkyl and $C_2$—$C_6$ alkenyl;

with the provisos, that,

(i) if Ar is a whether or not substituted para-phenylene group, and R is a whether or not substituted cyclohexyl group, or a saturated bi- or polycyclic hydrocarbyl group having 8—14 carbon atoms, then B does not represent an oxygen atom; and

(ii) if B is an oxygen atom, a —$CO_2$— group or an —NH—$CO_2$— group, then Ar is an unsubstituted para-phenylene group or para-pyridylene group.

These compounds have an interesting insecticidal and acaricidal activity as will become apparent from the examples.

Benzoylurea compounds of the general formulae

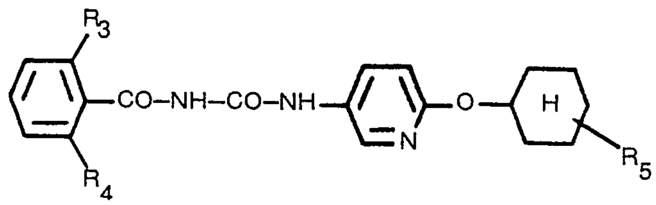

(II)

or

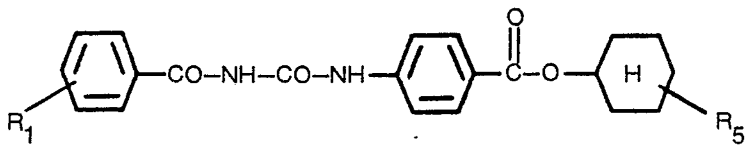

(III)

wherein

$R_1$ has the above meaning,

$R_3$ is a hydrogen atom or a halogen atom,

$R_4$ is a halogen atom, and

$R_5$ represents 1—3 alkyl or alkenyl groups having respectively 1—6 and 2—6 carbon atoms, are to be preferred on account of their high insecticidal activities.

Various compounds of the invention may occur in two stereoisomers, namely the *d*- and the *l*-form, while of course mixtures of these stereoisomers are also possible. If desired, the stereoisomers can be separated from each other by methods known for this purpose, but for practical considerations the use of a sterically pure starting material is to be preferred for the preparation of one of the stereoisomers in a pure form.

Examples of new benzoylurea compounds according to the invention are:

(1) N-(2-chlorobenzoyl)-N′-(2-*l*-menthyloxy-5-pyridyl)urea,
(2) N-(2,6-difluorobenzoyl)-N′-(2-*l*-menthyloxy-5-pyridyl)urea,
(3) N-(benzoyl-N′-(4-*dl*-menthyloxycarbonylphenyl)urea,
(4) N-(2-chlorobenzoyl)-N′-(4-*dl*-menthyloxycarbonylphenyl)urea,
(5) N-(2,6-difluorobenzoyl)-N′-(4-*dl*-menthyloxycarbonylphenyl)urea,
(6) N-(2-chlorobenzoyl)-N′-(4-*dl*-menthyloxycarbonylaminophenyl)urea,
(7) N-(2,6-difluorobenzoyl)-N′-(4-*dl*-menthyloxycarbonylaminophenyl)urea,
(8) N-(2,6-difluorobenzoyl)-N′-(4-cyclododecyloxyphenyl)urea,
(9) N-(2,6-difluorobenzoyl)-N′-(4-myrtenyloxyphenyl)urea,
(10) N-(2,6-difluorobenzoyl)-N′-[4-(2-isopropylcyclohexyloxycarbonyl)phenyl]urea,
(11) N-(2-chlorobenzoyl)-N′-[4-(2-isopropylcyclohexyloxycarbonyl)phenyl]urea,
(12) N-(2-chlorobenzoyl)-N′-[4-(3,5,5-trimethylcyclohexenyl-2-yloxy)phenyl]urea,
(13) N-(2,6-difluorobenzoyl)-N′-[4-(3,5,5-trimethylcyclohexen-2-yloxy)phenyl]urea,
(14) N-(2-chlorobenzoyl)-N′-[4-(1-cyclohexyl-n-butoxy)phenyl]urea,
(15) N-(2,6-difluorobenzoyl)-N′-[4-(1-cyclohexyl-n-butoxy)phenyl]urea,
(16) N-(2-chlorobenzoyl)-N′-[4-(1-cyclohexyl-2-methyl-n-propoxy)phenyl]urea,
(17) N-(2,6-difluorobenzoyl)-N′-[4-(1-cyclohexyl-2-methyl-n-propoxy)phenyl]urea,
(18) N-(2-chlorobenzoyl)-N′-[3,5-dichloro-4-(1-cyclohexyl-n-butoxy)phenyl]urea, and
(19) N-(2,6-difluorobenzoyl)-N′-[3,5-dichloro-4-(1-cyclohexyl-n-butoxy)phenyl]urea.

Related benzoylurea compounds are the subject of the non-prepublished European patent application 83201863.4.

The substances according to the invention may be used for the control of insects in agriculture and horticulture, in forests and in surface water, as well as for the protection of textile against attack by, for example, moths and carpet beetles, against insects in stocks, for example, in stored cereals, and against insects in the veterinary and medical-hygienic sectors.

The substances according to the invention may also be used for the control of insects living in the manure of warm-blooded animals, such as cows, pigs and hens. For this application the active compounds can be administered orally to the animals, for example, mixed through the food, so that they land in the manure after some time ("through-feeding").

The compounds according to the invention are particularly active against larvae and eggs of insects. In principle, the compounds may be used against all insects mentioned in Pestic. Sci. *9*, 373—386 (1978).

For practical pesticidal application the substances in accordance with the invention are usually processed in compositions. In such compositions the active substance is mixed with solid carrier material or dissolved or dispersed in liquid carrier material, if desired in combination with auxiliary substances, for example, emulsifiers, wetting agents, dispersible agents and stabilizers.

Examples of compositions according to the invention are aqueous solutions and dispersions, oily solutions and oily dispersions, solutions in organic solvents, pastes, dusting powders, dispersible powders, miscible oils, granules, pellets and aerosol compositions.

Dispersible powders, pastes and miscible oils are compositions in concentrate form which are diluted prior to or during use.

The invert emulsions and solutions in organic solvents are mainly used in air application, namely when large areas are treated with a comparatively small quantity of composition. The invert emulsion can be prepared shortly before or even during spraying in the spraying apparatus by emulsifying water in an oily solution or an oily dispersion of the active substance. The solutions of the active substance in organic solvents may be provided with a phytotoxicity-reducing substance, for example, wool fat, wool fatty acid or wool fatty alcohol.

A few forms of composition will be described in more detail hereinafter by way of example.

Granular compositions are prepared by taking up, for example, the active substance in a solvent or dispersing it in a diluent and impregnating the resulting solution/suspension, if desired in the presence of a binder, or granular carrier material, for example porous granules (for example pumice and attaclay), mineral non-porous granules (sand or ground marl), organic granules (for example, dried coffee grounds, cut tobacco stems or ground corncobs). A granular composition can also be prepared by compressing the active substance together with powdered minerals in the presence of lubricants and binders and disintegrating the compressed product to the desired grain size and sieving it. Granular compositions can be prepared in a different manner by mixing the active substance in powder form with powdered fillers, and glomulating the mixture then with liquid to the desired particle size.

Dusting powders can be obtained by intimately mixing the active substance with an inert solid powdered carrier material, for example, talcum.

Dispersible powders are prepared by mixing 10 to 80 parts by weight of a solid inert carrier, for example kaolin, dolomite, gypsum, chalk, bentonite, attapulgite, colloidal $SiO_2$ or mixtures of these and similar substances, with 10 to 80 parts by weight of the active substance, 1 to 5 parts by weight or a dispersing agent, for example the lignine sulphonates or alkylnaphthalene sulphonates known for this purpose, preferably also 0.5 to 5 parts by weight of a wetting agent, for example, fatt alcohol sulphates, alkyl aryl sulphonates, fatty acid condensation products, or polyoxyethylene compounds, and finally, if desired, other additives.

For the preparation of miscible oils the active compound is dissolved in a suitable solvent which preferably is poorly water-miscible, and one or more emulsifiers are added to this solution. Suitable solvents are, for example, xylene, toluene, petroleum distillates which are rich in aromates, for example, solvent naphtha, distilled tar oil and mixtures of these liquids. As emulsifiers may be used, for example, polyoxyethylene compounds and/or alkyl aryl sulphonates. The concentration of the active compound in these miscible oils is not restricted to narrow limits and may vary, for example, between 2 and 50% by weight.

In addition to a miscible oil may also be mentioned as a liquid and highly concentrated primary composition a solution of the active substance in a readily water-miscible liquid, for example, a glycol, a glycol ether, dimethylformamide, or N-methylpyrrolidone, to which solution a dispersing agent, and if desired, a surface-active substance has been added. When diluting with water shortly before or during spraying, an aqueous dispersion of the active substance is then obtained.

An aerosol composition according to the invention is obtained in the usual manner by incorporation the active substance, if desired in a solvent, in a volatile liquid to be used as a propellant, for example, a mixture of chlorine-fluorine derivatives of methane and ethane, a mixture of lower hydrocarbons, dimethyl ether, or gases such as carbon dioxide, nitrogen and nitrous oxide.

Fumigating candles or fumigating powders, i.e. compositions which, while burning, can generate a pesticidal smoke, are obtained by taking up the active substance in a combustible mixture which may contain as a fuel a sugar or a wood, preferably in a ground form, a substance to maintain combustion, for example, ammonium nitrate or potassium chlorate, and furthermore a substance to delay combustion, for example, kaolin, bentonite and/or colloidal silicic acid.

In addition to the above-mentioned ingredients, the agents according to the invention may also contain other substances known for use in this type of agents. For example a lubricant, e.g., calcium stearate or magnesium stearate, may be added to a dispersible powder or a mixture to be granulated. "Adhesives", for example, polyvinylalcohol, cellulose derivatives or other colloidal materials, such as casein, may also be added so as to improve the adhesion of the pesticide to the crop. Furthermore, a substance may be added to reduce the phytotoxicity of the active substance, carrier material or auxiliary substance, for example, wool fat or wool fatty alcohol.

Pesticidal compounds known *per se* may also be incorporated in the compositions according to the invention. As a result of this the activity spectrum of the composition is widened and synergism may occur.

For use in such a combination composition are to be considered the following known insecticidal, acaricidal and fungicidal compounds:

*Insecticides, for example:*

1. organic chlorine compounds, for example 6,7,8,9,10,10-hexachloro-1,5,5a,6,9a-hexahydro-6,9-methano-2,4,3-benzo[e]-dioxathiepine-3-oxide;
2. carbamates, for example, 2-dimethylamino-5,6-dimethylpyrimidin-4-yl dimethyl carbamate and 2-isopropoxyphenyl methylcarbamate;
3. di(m)ethylphosphates, for example, 2-chloro-2-diethylcarbamoyl-1-methylvinyl-, 2-methoxycarbonyl-1-methylvinyl-, 2-chloro-1-(2,4-dichlorophenyl)vinyl-, and 2-chloro-1-(2,4,5-trichlorophenyl)vinyl di(m)ethylphosphate;
4. O,O-di(m)ethyl phosphorothioates, for example, O(S)-2-methylthioethyl-, S-2-ethylsulphinylethyl-, S-2-(1-methylcarbamoylethylthio)ethyl-, O-4-bromo-2,5-dichlorophenyl-, O-3,5,6-trichloro-2-pyridyl-, O-2-isopropyl-6-methylpyrimidin-4-yl-, and O-4-nitrophenyl O,O-di(m)ethyl phosphorothioate;
5. O,O-di(m)ethyl phosphorodithioates, for example, S-methylcarbamoylmethyl-, S-2-ethylthioethyl-, S-(3,4-dihydro-4-oxobenzo[d]-1,2,3-triazin-3-ylmethyl)-, S-1,2-di(ethoxycarbonyl)ethyl-, S-6-chloro-2-oxo-

benzoxazolin-3-ylmethyl-, and S-2,3-dihydro-5-methoxy-2-oxo-1,3,4-thiadiazol-3-ylmethyl O,O-di(m)ethyl-phosphorodithioate;

6. phosphonates, for example, dimethyl 2,2,2-trichloro-1-hydroxyethylphosphonate;
7. natural and synthetic pyrethroids;
8. amidines, for example, N'-(2-methyl-4-chlorophenyl)-N,N-dimethylformamidine;
9. microbial insecticides, such as Bacillus thuringiensis;
10. carbamoyl-oximes, such as S-methyl N-(methylcarbamoyloxy) thioacetamidate; and
11. other benzoylurea compounds, such as N-(2,6-difluorobenzoyl)-N'-(4-chlorophenyl)urea.

*Acaricides, for example:*

1. organic tin compounds, for example, tricyclohexyl tin hydroxide and di[tri-(2-methyl-2-phenylpropyl)tin]oxide;
2. organic halogen compounds, for example isopropyl 4,4'-dibromobenzilate, 2,2,2-trichloro-1,1-di(4-chlorphenyl)ethanol and 2,4,5,4'-tetrachlorodiphenyl sulphone;
3. synthetic pyrethroids,

and furthermore: 3-chloro-α-ethoxyimino-2,6-dimethoxybenzyl benzoate and O,O-dimethyl S-methylcarbamoyl methyl phosphorothioate.

*Fungicides, for example:*

1. organic tin compounds, for example, triphenyl tin hydroxide and triphenyl tin acetate;
2. alkylene bisdithiocarbamates, for example, zinc ethylenebisdithiocarbamate and manganese ethylene bisdithiocarbamate;
3. 1-acyl- or 1-carbamoyl-N-benzimidazole (-2) carbamates and 1,2-bis (3-alkoxycarbonyl-2-thiureido)-benzene;

and furthermore 2,4-dinitro-6-(2-octylphenylcrotonate); 1-[bis(dimethylamino)phosphoryl]-3-phenyl-5-amino-1,2,4-triazole; N-trichloromethylthiophthalimide; N-trichloromethylthiotetrahydrophthalimide; N-(1,1,2,2-tetrachloroethylthio)-tetrahydrophthalimide; N-dichlorofluoromethylthio-N-phenyl-N,N'-dimethyl-sulphamide; tetrachloroisophthalonitrile; 2-(4'-thiazolyl)benzimidazole; 5-butyl-2-ethylamino-6-methylpyrimidine-4-yl-dimethylsulphamate; 1-(4-chlorophenoxy)-3,3-dimethyl-1(1,2,4-triazole-1-yl)-2-butanone; α-(2-chlorophenyl)-α-(4-chlorophenyl)-5-pyrimidinemethanol; 1-(isopropylcarbamoyl)-3-(3,5-dichloro-phenyl) hydantoin; N-(1,1,2,2-tetrachloroethylthio)-4-cyclohexene-1,2-carboximide; N-trichloromethyl-mercapto-4-cyclohexene-1,2-dicarboximide; N-tridecyl-2,6-dimethylmorpholine; metal salts of ethyl-phosphite; and N-(2,6-dimethylphenyl-N-methoxyacetyl)alanine methylester; or mixtures of these compounds.

The dosage of the insecticidal composition according to the invention desired for practical application will, of course, depend on various factors, for example, application area, selected active material, composition form, nature and extent of the infection, and the weather conditions.

In general it holds that favourable results are achieved with a dosage which corresponds to 1 to 5,000 g of the active substance per hectare.

For the above-described "through-feeding" the active substance is mixed through the food in a quantity which is effective for insecticidal applications.

The compounds according to the invention are novel substances which can be prepared in a manner known *per se* for related compounds.

For example, the compounds according to the invention can be prepared by reacting a substituted aniline of the general formula

$$R—B—Ar—NH_2$$

wherein R, B and Ar have the above meanings,
with a benzoylisocyanate of the general formula

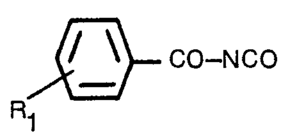

wherein $R_1$ also has the above meaning.

The novel compounds according to the invention can also be prepared by reacting a substituted benzamide of the general formula

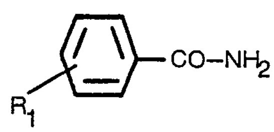

wherein $R_1$ has the above meaning,

with an isocyanate of the general formula

R—B—Ar—NCO

wherein $R_1$, B and Ar also have the above meanings.

The above reactions are preferably carried out in the presence of an organic solvent, for example, an aromatic hydrocarbon, an alkyl halide, a cyclic or non-cyclic dialkyl ether, or acetonitrile, at a reaction temperature between 0°C and the boiling-point of the solvent used.

Although the above-indicated methods of preparation are best suitable, the novel compounds may also be prepared in a different way, for example, as described in the above-mentioned Netherlands Patent Application 7105350 or according to the methods described in Netherlands Patent Applications 7806678 or 8005588.

The invention will now be described in greater detail with reference to the ensuing specific examples.

## Example I

Preparation of N-(2,6-difluorobenzoyl)-N'-(4-*dl*-menthyloxycarbonylphenyl)urea. (5)

0.92 g of 2,6-difluorobenzoylisocyanate were added to a solution of 1.37 g of 4-*dl*-methyloxycarbonylaniline in 50 ml of dry diethyl ether while stirring at room temperature.

After stirring for an additional half hour and adding 50 ml of petroleum ether (40—60) the formed precipitate was sucked off, washed with petroleum ether and dried. The desired product was obtained in a yield of 2.0 g; melting-point 169—170°C. The starting aniline was obtained from the corresponding nitro compound by reduction with hydrogen under the influence of Raney nickel as a catalyst, ethyl acetate being used as a solvent. 1-Nitro-4-*dl*-menthyloxycarbonylbenzene was prepared by reaction of *dl*-menthol with 4-nitrobenzoylchloride.

In a corresponding manner, in which if desired, instead of diethyl ether acetonitrile was used as a solvent for the urea-formation, the following compounds were prepared; the compound numbers correspond with the numbers given before in the specification:

| compound no. | melting point | compound no. | melting point |
|---|---|---|---|
| 1 | 174-176°C | 11 | 162-163°C |
| 2 | 144-147°C | 12 | 189-190°C |
| 3 | 222-223°C | 13 | 182-183°C |
| 4 | 195-196°C | 14 | 54- 57°C |
| 5 | 169-170°C | 15 | 123-130°C |
| 6 | 193-196°C | 16 | 133-141°C |
| 7 | 196-202°C | 17 | 116-123°C |
| 8 | 172-184°C | 18 | 154-166°C |
| 9 | 168-169°C | 19 | 98-110°C |
| 10 | 203-205°C | | |

## Example II

(a) Preparation of a solution of an active substance, namely N-(2,6-difluorobenzoyl)-N'-(4-*l*-menthyloxy-5-pyridyl)urea (2), in a water-miscible liquid ("liquid").

10 mg of the above active substance were dissolved in a mixture of 10 ml of isophorone and approximately 70 ml of dimethylformamide, after which polyoxyethylene glycol ricinly ether as an emulsifier was added in a quantity of 10 g.

The other active substances were processed to 10% or 20% "liquids" in a corresponding manner.

In a corresponding manner, "liquids" were obtained in N-methylpyrrolidone, dimethylformamide, and a mixture of N-methylpyrrolidone and isophorone as solvents.

(b) Preparation of a solution of the active substance in an organic solvent.

200 mg of the active substance to be investigated were dissolved in 1,000 ml of acetone in the presence of 1.6 g of nonylphenolpolyoxyethylene. After pouring in water, this solution may be used as a spraying liquid.

(c) Preparation of an emulsifiable concentrate of the active substance.

10 g of the active substance to be investigated were dissolved in a mixture of 15 ml of isophorone and 70 ml of xylene; 5 g of a mixture of a polyoxyethylene sorbitan ester and an alkyl benzene sulphonate as an emulsifier were added to this solution.

(d) Preparation of a dispersible powder (W.P.) of the active substance.

25 g of the active substance to be investigated were mixed with 68 g of kaolin in the presence of 2 g of sodium butylnaphthalene sulphonate and 5 g of lignine sulphonate.

(e) Preparation of a suspension concentrate (flowable) of the active substance.

A mixture of 10 g of active substance, 2 g of lignine sulphonate and 0.8 g of sodium alkylsulphate were supplied with water till a total amount of 100 ml.

(f) Preparation of a granule of the active substance. 7.5 g of active substance, 5 g of sulphite lye and 87.5 g of ground dolomite were mixed, after which the resulting mixture was processed to a granular composition by means of the so-called compacting method.

## Example III

Young Brussels sprouts plants, approximately 15 cm high, wree sprayed till run-off with compositions obtained according to Example II (b) in various concentrations; to these compositions had been added in addition approximately 250 mg of an alkylated phenol polyoxyethylene compound (Citowett) per litre. After the plants had dried, they were placed in cylinders of plexiglass and then infected with 5 larvae of *Pieris brassicae* (cabbage white bufferfly) in the third larval stage (L3). The cylinders were then covered with a gauze and stored, an alternating light-dark cycle of 16 hours light and 8 hours dark being used; temperature in the light 24°C, rel. humidity (RH) 70%; temperature in the dark 19°C. 80—90% RH. After 5 days the mortality percentage of the larvae was established. Each experiment was carried out in triplicate. The average results of the experiments are recorded in Table A below.

The meanings of the symbols in the table are as follows:

+ = 90—100% mortality
± = 50—90% mortality
− = <50% mortality

N-(2-chlorobenzoyl)-N'-4-(1-phenylcyclohexyloxy)phenyl urea ("known") has been included in the tests by way of comparison.

TABLE A

Insecticidal activity against larvae (L3) of Pieris brassicae

| Comp. no. | Concentration in mg of active substance per litre | | | | | | | | |
|---|---|---|---|---|---|---|---|---|---|
| | 300 | 100 | 30 | 10 | 3 | 1 | 0.3 | 0.1 | 0.03 |
| (1) | + | + | + | + | + | + | - | | |
| (2) | + | + | + | + | + | + | + | ± | - |
| (3) | + | + | + | + | + | | | | |
| (4) | + | + | + | + | + | | | | |
| (5) | + | + | + | + | + | + | + | | |
| (6) | + | + | + | + | + | - | | | |
| (7) | + | + | + | + | + | ± | - | | |
| (8) | + | + | + | + | + | + | + | | |
| (10) | + | + | + | + | + | + | + | | |
| (12) | + | + | + | + | + | + | + | | |
| (13) | + | + | + | + | + | + | + | | |
| "known" | + | + | - | | | | | | |

Note: If the test results do not finish with a "−" sign, the tests have not been completed.

Liquid insecticidal compositions are used in practice in quantities of approximately 1,000 litres per hectare. The coverage of the plants with the composition, however, is considerably less good in practice than in a laboratory of glasshouse experiment as described herein before. It has hence been found that in practice the dose is to be increased by a factor 10 to obtain the same efficiency.

So in practical applications the above quantities with insecticidal activity correspond to approximately 3 to approximately 3,000 g of active substance per hectare.

Example IV

20 Larvae of *Aedes aegypti* (yellow-fever mosquito) were placed in various concentrations of aqueous suspensions of the active substances obtained according to example II (e). The suspensions were kept at a temperature of 25°C for 10 days, during which incubation period the larvae were fed with an aqueous suspension of powdered brown bread and yeast. After 10 days the mortality percentage was determined taking the natural mortality into account. The results of the experiment are recorded in Table B. The meaning of the symbols is the same as in Example III.

TABLE B

Insecticidal activity against larvae (L1) of Aedes aegypti

| Comp. no. | Concentration in mg of active substance per litre | | | | | | |
|---|---|---|---|---|---|---|---|
| | 1 | 0.3 | 0.1 | 0.03 | 0.01 | 0.003 | 0.001 |
| (2) | + | + | + | + | + | + | - |
| (7) | + | + | + | - | | | |
| (8) | + | + | + | + | + | ± | - |
| (12) | + | + | + | ± | ± | - | |
| (13) | + | + | + | + | + | ± | - |
| (15) | + | + | + | + | - | | |
| (17) | + | + | + | + | - | | |
| (18) | + | + | + | + | - | | |
| (19) | + | + | + | + | - | | |

Example V

The growth tops of broad beans were removed in such a way that four well developed leaves remained. The plants were sprayed till run-off with compositions obtained according to Example II (b) in various concentrations; to these compositions had moreover been added approximately 250 mg of Citowett per litre. After the plants had dried, they were placed in perspex cylinders and then infected with 5 larvae of *Spodoptera littoralis* (Egyptian cotton leafworm) in the third larval stage (L3). The cylinders were then covered with a gauze and then stored as described in Example III. After 5 days the mortality percentage of the larvae was established. Each experiment was carried out in triplicate. The average results of the experiments are recorded in table C. The meanings of the symbols are the same as in Example III.

TABLE C

Insecticidal activity against larvae (L3) of Spodoptera littoralis

| Comp. no. | Concentration in mg of active substance per litre | | | | | | |
|---|---|---|---|---|---|---|---|
| | 300 | 100 | 30 | 10 | 3 | 1 | 0.3 |
| (1) | + | + | + | + | + | ± | - |
| (2) | + | + | + | + | + | ± | - |
| (3) | + | + | + | + | + | - | |
| (4) | + | + | + | + | + | + | - |
| (5) | + | + | + | + | + | ± | |
| (6) | + | + | + | ± | ± | - | |
| (7) | + | + | + | + | - | | |
| (8) | + | + | + | ± | ± | | |
| (9) | + | + | ± | | | | |
| (10) | + | + | + | + | + | + | ± |
| (13) | + | + | + | + | ± | ± | - |

In practice the above quantities with insecticidal activity correspond to approx. 10 to approx. 3,000 g of active substance per litre.

Example VI

Young potato plants, approx. 15 cm high, were sprayed with the compositions obtained according to Example II (b) in various concentrations; in addition approx. 250 mg of Citowett per litre had been added to these compositions. After the plants had dried, plexiglass cylinders were placed over the plants. The plants were then infected with 10 larvae of *Leptinotarsa decemlineata* (Colorado potato beetle) in the third larval stage (L3). The infected plants were stored as indicated in Example III. After 5 days the mortality percentage of the larvae was established. The experiments have been carried out in triplicate. The average results of the experiments are recorded in table D below. The meanings of the symbols are the same as in Example III. The same "known" compound was used as in Example III.

TABLE D

Insecticidal activity against larvae (L3) of Leptinotarsa decemlineata

| Comp. no. | Concentration in mg of active substance per litre | | | | |
|---|---|---|---|---|---|
| | 300 | 100 | 30 | 10 | 3 |
| (2) | + | + | + | + | - |
| (3) | + | + | - | | |
| (4) | + | + | + | ± | - |
| (5) | + | + | + | + | ± |
| (6) | + | + | + | - | |
| (7) | + | + | + | ± | - |
| (8) | + | + | + | + | ± |
| (9) | + | + | + | | |
| (10) | + | + | + | + | + |

TABLE D (cont.)

Insecticidal activity against larvae (L3) of Leptinotarsa decemlineata

| Comp. no. | Concentration in mg of active substance per litre | | | | | | |
|---|---|---|---|---|---|---|---|
| | 300 | 100 | 30 | 10 | 3 | 1 | 0.3 |
| (12) | + | + | + | + | + | + | ± |
| (13) | + | + | + | + | ± | + | ± |
| (14) | + | + | + | - | | | |
| (15) | + | + | + | + | - | | |
| (17) | + | + | + | - | | | |
| (18) | + | + | + | + | ± | | |
| (19) | + | + | + | + | + | | |
| "known" | - | | | | | | |

In practice the above quantities with insecticidal activity correspond with approx. 30 to approx. 3000 grams of active substance per hectare.

Example VII

Acaricidal activity against larvae and eggs of Tetranychus cinnabarinus.

Dwarf French bean plants (Phaseolus vulgaris) having two well developed leaves were infected with *Tetranychus cinnabarinus* (carnation spider mite) by placing a given number of adult female mites on the plants. Two days after the infection the plants with the adult mites present thereon were sprayed till run-off with compositions obtained according to Example II (b) in various concentrations; to these compositions had moreover been added approximately 150 mg of an alkylated phenolpolyoxyethylene compound (Citowett) per litre. 5 Days after spraying, the adult insects were removed from the plants. The plants were stored for 2 weeks in a space with controlled temperature (T) and relative humidity (RH), an alternating light-dark cycle being used of 16 hours light and 8 hours dark. Light: T approximately 24°C, RH approximately 70%; dark: T approximately 19°C, RH 80—90%. The reduction of the population, i.e. the mortality of the larvae and eggs as compared with plants not treated with chemicals was established. The experiment was carried out in triplicate. When using a composition which contained compound (15) as the active substance, a reduction of the population with approx. 100% as compared with plants not treated with chemicals was found.

**Claims for the Contracting States: BE CH DE FR GB IT LI LU NL SE**

1. Benzoylurea compounds of the general formula

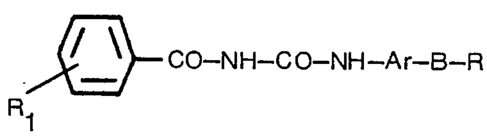 (I)

wherein

$R_1$ is a hydrogen atom or represents 1 or 2 halogen atoms;

Ar is a para-phenylene group or a para-pyridylene group, which groups may be substituted with 1 or 2 substituents selected from the group consisting of chlorine, methyl and trifluoromethyl;

B is an oxygen atom or represents a group having the formula

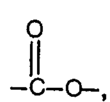, 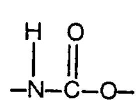 or 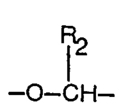

wherein $R_2$ is a hydrogen atom or an alkyl group having 1—4 carbon atoms; and

R is a cycloalkyl group or a cycloalkenyl group having 6—12 carbon atoms, which groups may be substituted with 1—3 substituents selected from the group consisting of $C_1$—$C_6$ alkyl and $C_2$—$C_6$ alkenyl, or wherein

R is a bi- or polycyclic, saturated or unsaturated, non-aromatic hydrocarbyl group, having 8—14 carbon atoms and, if desired, substituted with 1—3 substituents selected from the group consisting of $C_1$—$C_6$ alkyl and $C_2$—$C_6$ alkenyl;

with the provisos, that,

(i) If Ar is a whether or not substituted para-phenylene group, and R is a whether or not substituted cyclohexyl group, or a saturated bi- or polycyclic hydrocarbyl group having 8—14 carbon atoms, then B does not represent an oxygen atom; and

(ii) if B is an oxygen atom, a —$CO_2$— group or an —NH—$CO_2$— group, then Ar is an unsubstituted para-phenylene group or para-pyridylene group.

2. Benzoylurea compounds as claimed in Claim 1 of the general formula

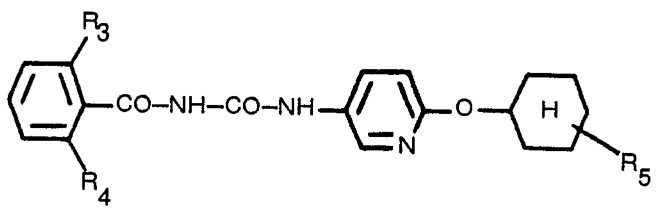

(II)

wherein

$R_3$ is a hydrogen atom or a halogen atom,

$R_4$ is a halogen atom, and

$R_5$ represents 1—3 alkyl or alkenyl groups having respectively 1—6 and 2—6 carbon atoms.

3. Benzoylurea compounds as claimed in Claim 1 of the general formula

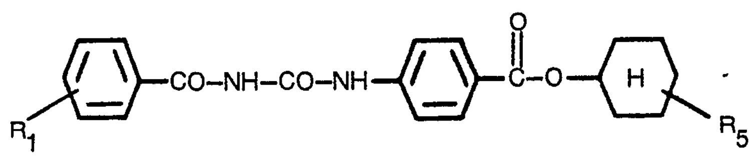

(III)

wherein

$R_1$ has the meaning given in Claim 1, and

$R_5$ has the meaning given in Claim 2.

4. A method of preparing new benzoylurea compounds, characterized in that compounds of the general formula I, wherein $R_1$, Ar, B and R have the meanings given in Claim 1, are prepared by reacting a compound of the general formula

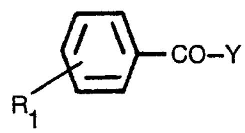

wherein

$R_1$ has the meaning given in Claim 1, and

Y is an amino group or an isocyanato group, with a compound of the general formula

R—B—Ar—Z

wherein

R, B and Ar have the meanings given in Claim 1, and Z is an isocyanato group or an amino group, with the provisos, that if Y is an amino group then Z is an isocyanato group, and if Y is an isocyanato group then Z is an amino group.

5. A composition having insecticidal and acaricidal activity, characterized in that, in addition to a liquid or solid inert carrier material, the composition comprises a compound of the general formula I, wherein $R_1$, Ar, B and R have the meanings given in Claim 1.

6. A composition as claimed in Claim 5, characterized in that the active constituent is a compound of the general formula II, wherein $R_3$, $R_4$ and $R_5$ have the meanings given in Claim 2.

7. A composition as claimed in Claim 5, characterized in that the active constituent is a compound of the general formula III, wherein $R_1$ has the meaning given in Claim 1, and $R_5$ has the meaning given in Claim 2.

8. A method of preparing a composition having insecticidal and acaricidal activity, characterized in that a compound of the general formula I, wherein $R_1$, Ar, B and R have the meanings given in Claim 1, is

included into a liquid or solid inert carrier material, if desired while adding other pesticidal compounds, artificial manures and/or auxiliary substances.

9. A method of controling insects and/or mites characterized in that the infected area is treated with a composition as claimed in any of the claims 5 to 7 in a dosage from 1 to 5,000 g of active substance per hectare.

**Claims for the Contracting State: AT**

1. A method of preparing new benzoylurea compounds, characterized in that compounds of the general formula

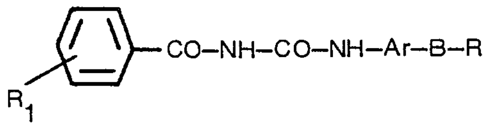 (I)

wherein

$R_1$ is a hydrogen atom or represents 1 or 2 halogen atoms;

Ar is a para-phenylene group or a para-pyridylene group, which groups may be substituted with 1 or 2 substituents selected from the group consisting of chlorine, methyl and trifluoromethyl;

B is an oxygen atom or represents a group having the formula

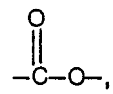, 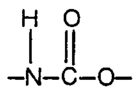 or 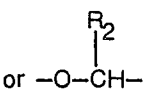

wherein $R_2$ is a hydrogen atom or an alkyl group having 1—4 carbon atoms; and

R is a cycloalkyl group or a cycloalkenyl group having 6—12 carbon atoms, which groups may be substituted with 1—3 substituents selected from the group consisting of $C_1$—$C_6$ alkyl and $C_2$—$C_6$ alkenyl, or wherein

R is a bi- or polycyclic, saturated or unsaturated, non-aromatic hydrocarbyl group, having 8—14 carbon atoms and, if desired, substituted with 1—3 substituents selected from the group consisting of $C_1$—$C_6$ alkyl and $C_2$—$C_6$ alkenyl;

with the provisos, that,

(i) If Ar is a whether or not substituted para-phenylene group, and R is a whether or not substituted cyclohexyl group, or a saturated bi- or polycyclic hydrocarbyl group having 8—14 carbon atoms, then B does not represent an oxygen atom; and

(ii) if B is an oxygen atom, a —$CO_2$— group or an —NH—$CO_2$— group, then Ar is an unsubstituted para-phenylene group or para-pyrilidene group,

are prepared by reacting a compound of the general formula

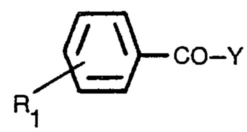

wherein

$R_1$ has the above meaning, and

Y is an amino group or an isocyanato group,

with a compound of the general formula

R—B—Ar—Z

wherein

R, B and Ar have the above meanings, and

Z is an isocyanato group or an amino group,

with the provisos, that if Y is an amino group then Z is an isocyanato group, and if Y is an isocyanato group then Z is an amino group.

2. A method as claimed in Claim 1, characterized in that benzoylurea compounds of the general formula

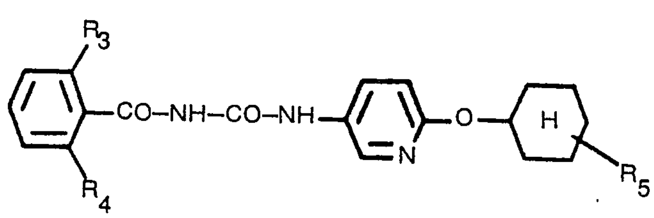 (II)

wherein

$R_3$ is a hydrogen atom or a halogen atom,

$R_4$ is a halogen atom, and

$R_5$ represents 1—3 alkyl or alkenyl groups having respectively 1—6 and 2—6 carbon atoms,

are prepared by a method as defined in Claim 1.

3. A method as claimed in Claim 1, characterized in that benzoylurea compounds of the general formula

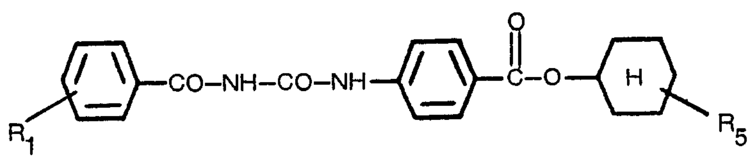

(III)

wherein

$R_1$ has the meaning given in Claim 1, and

$R_5$ has the meaning given in Claim 2,

are prepared by a method as defined in Claim 1.

4. A composition having insecticidal and acaricidal activity, characterized in that, in addition to a liquid or solid inert carrier material, the composition comprises a compound of the general formula I, wherein $R_1$, Ar, B and R have the meanings given in Claim 1.

5. A method as claimed in Claim 4, characterized in that the active constituent is a compound of the general formula II, wherein $R_3$, $R_4$ and $R_5$ have the meanings given in Claim 2.

6. A composition as claimed in Claim 4, characterized in that the active constituent is a compound of the general formula III, wherein $R_1$ has the meaning given in Claim 1, and $R_5$ has the meaning given in Claim 2.

7. A method of preparing a composition having insecticidal and acaricidal activity, characterized in that a compound of the general formula I, wherein $R_1$, Ar, B and R have the meanings given in Claim 1, is included into a liquid or solid inert carrier material, if desired while adding other pesticidal compounds, artificial manures and/or auxiliary substances.

8. A method of controlling insects and/or mites characterized in that the infected area is treated with a composition as claimed in any of the claims 4 to 6 in a dosage from 1 to 5,000 g of active substance per hectare.

**Patentansprüche für die Vertragsstaaten: BE CH DE FR GB IT LI LU NL SE**

1. Benzoylharnstoffverbindungen der allgemeinen Formel

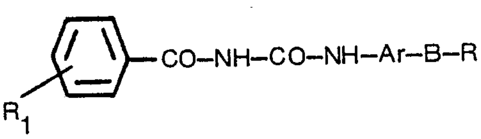

(I),

worin $R_1$ ein Wasserstoffatom ist oder 1 oder 2 Halogenatome darstellt; Ar eine p-Phenylengruppe oder eine p-Pyridylengruppe ist, welche Gruppe mit 1 oder 2 Substituenten ausgewählt aus der Gruppe bestehend aus Chlor, Methyl und Trifluormethyl substituiert sein können; B ein Sauerstoffatom ist oder eine Gruppe der Formel

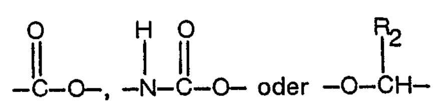

darstellt, wobei $R_2$ ein Wasserstoffatom oder eine Alkylgruppe mit 1 bis 4 Kohlenstoffatomen ist; und R eine Cycloalkylgruppe oder eine Cycloalkenylgruppe mit 6 bis 12 Kohlenstoffatomen ist, welche Gruppen mit 1 bis 3 Substituenten ausgewählt aus der Gruppe bestehend aus $C_1$—$C_6$-Alkyl und $C_2$—$C_6$-Alkenyl substituiert sein können, oder worin R eine bi- oder polycyclische, gesättigte oder ungesättigte, nichtaromatische Hydrocarbylgruppe mit 8 bis 14 Kohlenstoffatomen und, wenn gewünscht, substituiert mit 1 bis 3 Substituenten ausgewählt aus der Gruppe bestehend aus $C_1$—$C_6$-Alkyl und $C_2$—$C_6$-Alkenyl ist, mit den Maßgaben, daß,

(i) wenn Ar eine gegebenenfalls substiuierte p-Phenylengruppe ist und R eine gegebenenfalls substituierte Cyclohexylgruppe oder eine gesättigte bi- oder polycyclische Hydrocarbylgruppe mit 8 bis 14 Kohlenstoffatomen ist, B eine andere Bedeutung als ein Sauerstoffatom hat; und

(ii) wenn B ein Sauerstoffatom, eine —$CO_2$-Gruppe oder eine —NH—$CO_2$-Gruppe ist, Ar eine unsubstituierte p-Phenylengruppe oder eine p-Pyridylengruppe darstellt.

2. Benzoylharnstoffverbindungen wie in Anspruch 1 beansprucht, der allgemeinen Formel

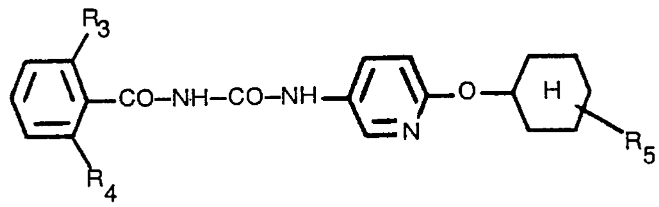

(II),

worin $R_3$ ein Wasserstoffatom oder ein Halogenatom ist, $R_4$ ein Halogenatom darstellt und $R_5$ bis 3 Alkyl- oder Alkenylgruppen mit 1 bis 6 bzw. 2 bis 6 Kohlenstoffatomen bedeutet.

3. Benzoylharnstoffverbindungen wie in Anspruch 1 beansprucht, der allgemeinen Formel

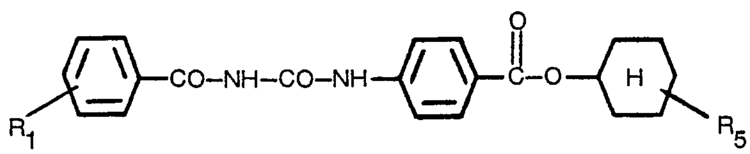

(III),

worin $R_1$ die in Anspruch 1 angegebene Bedeutung hat und $R_5$ die in Anspruch 2 angegebene Bedeutung hat.

4. Verfahren zum Herstellen neuer Benzoylharnstoffverbindungen der allgemeinen Formel (I), worin $R_1$, Ar, B und R die in Anspruch 1 angegebenen Bedeutungen haben, die hergestellt werden durch Umsetzen einer Verbindung der allgemeinen Formel

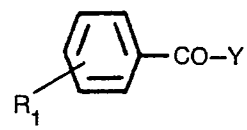 ,

worin $R_1$ die in Anspruch 1 angegebene Bedeutungen hat und Y eine Aminogruppe oder eine Isocyanatgruppe darstellt, mit einer Verbindung der allgemeinen Formel

$$R{-}B{-}Ar{-}Z \quad ,$$

worin R, B und Ar die in Anspruch 1 angegebenen Bedeutungen haben und Z eine Isocyanatgruppe oder eine Aminogruppe ist, mit den Maßgaben, daß, wenn Y eine Aminogruppe ist, Z eine Isocyanatgruppe darstellt, und wenn Y eine Isocyanatgruppe bedeutet, Z eine Aminogruppe ist.

5. Zusammensetzung mit insektizider und akarizider Wirksamkeit, dadurch gekennzeichnet, daß die Zusammensetzung zusätzlich zu einem flüssigen oder festen inerten Trägermaterial eine Verbindung der allgemeinen Formel (I), worin $R_1$, Ar, B und R die in Anspruch 1 angegebenen Bedeutungen haben, aufweist.

6. Zusammensetzung, wie in Anspruch 5 beansprucht, dadurch gekennzeichnet, daß der aktive Bestandteil eine Verbindung der allgemeinen Formel (II) ist, worin $R_3$, $R_4$ und $R_5$ die in Anspruch 2 angegebenen Bedeutungen haben.

7. Zusammensetzung, wie in Anspruch 5 beansprucht, dadurch gekennzeichnet, daß der aktive Bestandteil eine Verbindung der allgemeinen Formel (III) ist, worin $R_1$ die in Anspruch 1 angegebene Bedeutung hat und $R_5$ die in Anspruch 2 angegebene Bedeutung hat.

8. Verfahren zum Herstellen einer Zusammensetzung mit insektizider und akarizider Wirksamkeit, dadurch gekennzeichnet, daß eine Verbindung der allgemeinen Formel (I), worin $R_1$, Ar, B und R die in Anspruch 1 angegebene Bedeutung haben, einem flüssigen oder festen inerten Trägermaterial einverleibt wird, wenn gewünscht unter Zusatz anderer pestizider Verbindungen, künstlicher Düngemittel und/oder Hilfssubstanzen.

9. Verfahren zum Bekämpfen von Insekten und/oder Milben, dadurch gekennzeichnet, daß die befallene Fläche mit einer Zusammensetzung, wie in einem der Ansprüche 5 bis 7 beansprucht, in einer Dosierung von 1 bis 5000 g der aktiven Substanz pro ha behandelt wird.

**Patentansprüche für den Vertragsstaat: AT**

1. Verfahren zum Herstellen von neuen Benzoylharnstoffverbindungen, dadurch gekennzeichnet, daß Verbindungen der allgemeinen Formel

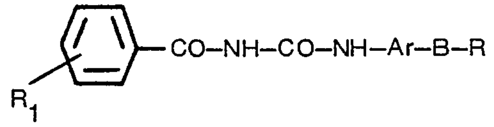

(I),

worin $R_1$ ein Wasserstoffatom ist oder 1 oder 2 Halogenatome darstellt; Ar eine p-Phenylengruppe oder eine p-Pyridylengruppe ist, welche Gruppe mit 1 oder 2 Substituenten ausgewählt aus der Gruppe bestehend aus Chlor, Methyl und Trifluormethyl substituiert sein können; B ein Sauerstoffatom ist oder eine Gruppe der Formel

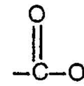, 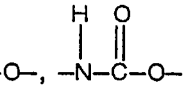 oder 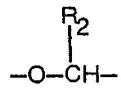

darstellt, wobei $R_2$ ein Wasserstofftom oder eine Alkylgruppe mit 1 bis 4 Kohlenstoffatomen ist; und R eine Cycloalkylgruppe oder eine Cycloalkenylgruppe mit 6 bis 12 Kohlenstoffatomen ist, welche Gruppen mit 1 bis 3 Substituenten ausgewählt aus der Gruppe bestehend aus $C_1$—$C_6$-Alkyl und $C_2$—$C_6$-Alkenyl substituiert sein können, oder worin R eine bi- oder polycyclische, gesättigte oder ungesättigte, nichtaromatische Hydrocarbylgruppe mit 8 bis 14 Kohlenstoffatomen und, wenn gewünscht, substituiert mit 1 bis 3 Substituenten ausgewählt aus der Gruppe bestehend aus $C_1$—$C_6$-Alkyl und $C_2$—$C_6$-Alkenyl ist, mit den Maßgaben, daß,

(i) wenn Ar eine gegebenenfalls substiuierte p-Phenylengruppe ist und R eine gegebenenfalls substituierte Cyclohexylgruppe oder eine gesättigte bi- oder polycyclische Hydrocarbylgruppe mit 8 bis 14 Kohlenstoffatomen ist, B eine andere Bedeutung als ein Sauerstoffatom hat; und

(ii) wenn B ein Sauerstoffatom, eine —$CO_2$-Gruppe oder eine —NH—$CO_2$-Gruppe ist, Ar eine unsubstituierte p-Phenylengruppe oder eine p-Pyridylengruppe darstellt,

hergestellt werden durch Umsetzen einer Verbindung der allgemeinen Formel

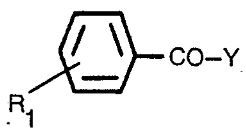 ,

worin $R_1$ die obige Bedeutung hat und Y eine Aminogruppe oder eine Isocyanatgruppe darstellt, mit einer Verbindung der allgemeinen Formel

R—B—Ar—Z ,

worin R, B und Ar die oben angegebenen Bedeutungen haben und Z eine Isocyanatgruppe oder eine Aminogruppe ist, mit den Maßgaben, daß, wenn Y eine Aminogruppe ist, Z eine Isocyanatgruppe darstellt, und wenn Y eine Isocyanatgruppe bedeutet, Z eine Aminogruppe ist.

2. Verfahren wie in Anspruch 1 beansprucht, dadurch gekennzeichnet, daß Benzoylharnstoffverbindungen der allgemeinen Formel

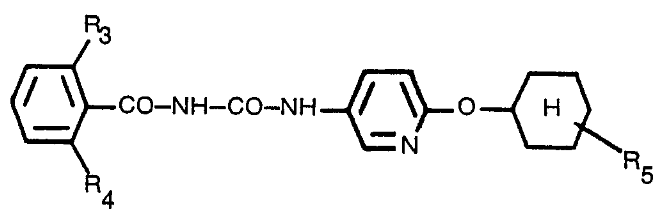 (II),

worin $R_3$ ein Wasserstoffatom oder ein Halogenatom ist, $R_4$ ein Halogenatom darstellt und $R_5$ 1 bis 3 Alkyl- oder Alkenylgruppen mit 1 bis 6 bzw. 2 bis 6 Kohlenstoffatomen bedeutet, nach einem Verfahren, wie in Anspruch 1 definiert, hergestellt werden.

3. Verfahren, wie in Anspruch 1 beansprucht, dadurch gekennzeichnet, daß Benzoylharnstoffverbindungen der allgemeinen Formel

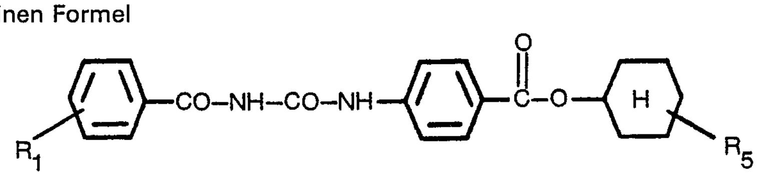 (III),

worin $R_1$ die in Anspruch 1 angegebene Bedeutung hat und $R_5$ die in Anspruch 2 angegebene Bedeutung hat, nach einem Verfahren, wie in Anspruch 1 definiert, hergestellt werden.

4. Zusammensetzung mit insektizider und akarizider Wirksamkeit, dadurch gekennzeichnet, daß die Zusammensetzung zusätzlich zu einem flüssigen oder festen inerten Trägermaterial eine Verbindung der allgemeinen Formel (I), worin $R_1$, Ar, B und R die in Anspruch 1 angegebenen Bedeutungen haben, aufweist.

5. Zusammensetzung, wie in Anspruch 4 beansprucht, dadurch gekennzeichnet, daß der aktive Bestandteil eine Verbindung der allgemeinen Formel (II) ist, worin $R_3$, $R_4$ und $R_5$ die in Anspruch 2 angegebenen Bedeutungen haben.

6. Zusammensetzung, wie in Anspruch 4 beansprucht, dadurch gekennzeichnet, daß der aktive Bestandteile eine Verbindung der allgemeinen Formel (III) ist, worin $R_1$ die in Anspruch 1 angegebene Bedeutung hat und $R_5$ die in Anspruch 2 angegebene Bedeutung hat.

7. Verfahren zum Herstellen einer Zusammensetzung mit insektizider und akarizider Wirksamkeit, dadurch gekennzeichnet, daß eine Verbindung der allgemeinen Formel (I), worin $R_1$, Ar, B und R die in Anspruch 1 angegebene Bedeutung haben, einem flüssigen oder festen inerten Trägermaterial einverleibt wird, wenn gewünscht unter Zusatz anderer pestizider Verbindungen, künstlicher Düngemittel und/oder Hilfssubstanzen.

8. Verfahren zum Bekämpfen von Insekten und/oder Milben, dadurch gekennzeichnet, daß die befallene Fläche mit einer Zusammensetzung, wie in einem der Ansprüche 4 bis 6 beansprucht, in einer Dosierung von 1 bis 5000 g der aktiven Substanz pro ha behandelt wird.

**Revendications pour les Etats contractants: BE CH DE FR GB IT LI LU NL SE**

1. Composés benzoylurées de formule générale

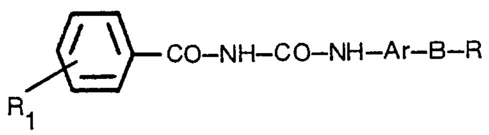
(I)

dans laquelle

$R_1$ est un atome d'hydrogène ou représente 1 ou 2 atomes d'halogène;

Ar est un groupe para-phénylène ou un groupe para-pyridylène, ces groupes pouvant être substitués par 1 ou 2 substituants choisis dans le groupe constitué par le chlore, le méthyle et le trifluorométhyle;

B est un atome d'oxygène ou représente un groupe de formule

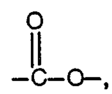, 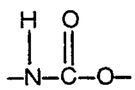 ou 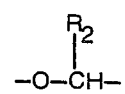

où $R_2$ est un atome d'hydrogène ou un groupe alkyle comportant 1—4 atomes de carbone; et

R est un groupe cycloalkyle ou un groupe cycloalcényle comportant 6—12 atomes de carbone, ces groupes pouvant être substitués par 1—3 substituants choisis dans le groupe constitué par les groupes alkyle en $C_1$—$C_6$ et alcényle en $C_2$—$C_6$, ou, dans la formule,

R est un groupe hydrocarboné non aromatique, bicyclique ou polycyclique, saturé ou insaturé, comportant 8—14 atomes de carbone, et éventuellement substitué par 1—3 substituants choisis dans le groupe constitué par les groupes alkyle en $C_1$—$C_6$ et alcényle en $C_2$—$C_6$;

à condition que

(i) si Ar est un groupe para-phénylène, substitué ou non substitué, et que R est un groupe cyclohexyle, substitué ou non substitué, ou un groupe hydrocarboné bicyclique ou polycyclique saturé comportant 8—14 atomes de carbone, B ne représente pas un atome d'oxygène; et

(ii) si B est un atome d'oxygène, un groupe —$CO_2$— ou un groupe —NH—$CO_2$—, Ar soit un groupe para-phénylène ou para-pyridylène non substitué.

2. Composés benzoylurées selon la revendication 1, de formule générale

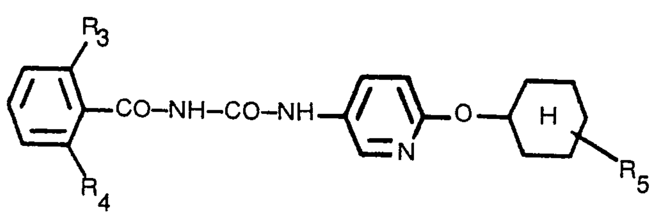
(II)

dans laquelle

$R_3$ est un atome d'hydrogène ou un atome d'halogène,

$R_4$ est un atome d'halogène, et

$R_5$ représente 1 à 3 groupes alkyle ou alcényle comportant respectivement 1—6 et 2—6 atomes de carbone.

3. Composés benzoylurées selon la revendication 1, de formule générale

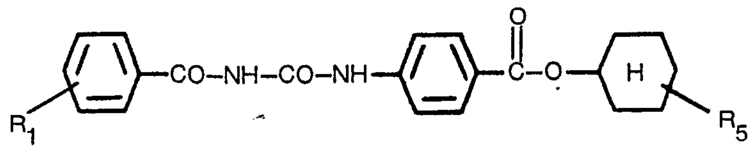

(III)

dans laquelle

$R_1$ a la signification indiquée dans la revendication 1, et

$R_5$ a la signification indiquée dans la revendication 2.

4. Procédé de préparation de nouveaux composés benzoylurées, caractérisé en ce que l'on prépare les composés de formule générale I, dans laquelle $R_1$, Ar, B et R ont les significations indiquées à la revendication 1, et faisant réagir un composé de formule générale

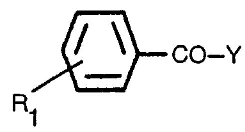

dans laquelle

$R_1$ a la signification indiquée dans la revendication 1, et

Y est un groupe amino ou un groupe isocyanato,

avec un composé de formule générale

R—B—Ar—Z

dans laquelle

R, B et Ar ont les significations indiquées à la revendication 1, et

Z est un groupe isocyanato ou un groupe amino,

à condition que, si Y est un groupe amino, Z soit un groupe isocyanato, et que si Y est un groupe isocyanato, Z soit un groupe amino.

5. Composition ayant une activité insecticide et acaricide, caractérisée en ce que, en plus d'un véhicule inerte liquide ou solide, la composition comprend un composé de formule générale I, dans laquelle $R_1$, Ar, B et R ont les significations indiquées à la revendication 1.

6. Composition selon la revendication 5, caractérisée en ce que le constituant actif est un composé de formule générale II, dans laquelle $R_3$, $R_4$ et $R_5$ ont les significations indiquées à la revendication 2.

7. Composition selon la revendication 5, caractérisée en ce que le constituant actif est un composé de formule générale III, dans laquelle $R_1$ a la signification indiquée à la revendication 1, et $R_5$ a la signification indiquée à la revendication 2.

8. Procédé de préparation d'une composition ayant une activité insecticide et acaricide, caractérisé en ce qu'un composé de formule générale I, dans laquelle $R_1$, Ar, B et R ont les significations indiquées à la revendication 1, est inclu dans un véhicule inerte liquide ou solide, éventuellement avec d'autres composés pesticides, des fumiers artificiels et/ou des substances auxiliaires.

9. Procédé de lutte contre les insectes et/ou les acariens, caractérisé en ce que l'on traite la zone infectée avec une composition selon l'une quelconque des revendications 5 à 7, en dose de 1 à 5000 g de substance active par hectare.

**Revendications pour l'Etat contractant: AT**

1. Procédé de préparation de nouveaux composés benzoylurées, caractérisé en ce que l'on prépare des composés de formule générale

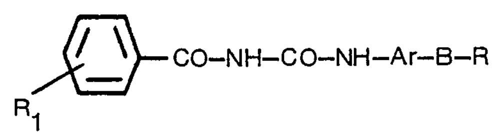

(I)

dans laquelle

$R_1$ est un atome d'hydrogène ou représente 1 ou 2 atomes d'halogène;

Ar est un groupe para-phénylène ou un groupe para-pyridylène, ces groupes pouvant être substitués par 1 ou 2 substituants choisis dans le groupe constitué par le chlore, le méthyle et le trifluorométhyle;

B est un atome d'oxygène ou représente un groupe de formule

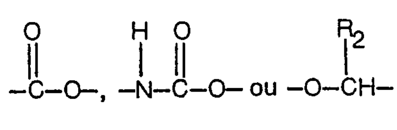

où $R_2$ est un atome d'hydrogène ou un groupe alkyle comportant 1—4 atomes de carbone; et

R est un groupe cycloalkyle ou un groupe cycloalcényle comportant 6—12 atomes de carbone, ces groupes pouvant être substitués par 1—3 substituants choisis dans le groupe constitué par les groupes alkyle en $C_1$—$C_6$ et alcényle en $C_2$—$C_6$, ou, dans la formule,

R est un groupe hydrocarboné non aromatique, bicyclique ou polycyclique, saturé ou insaturé, comportant 8—14 atomes de carbone, et éventuellement substitué par 1—3 substituants choisis dans le groupe constitué par les groupes alkyle en $C_1$—$C_6$ et alcényle en $C_2$—$C_6$;

à condition que

(i) si Ar est un groupe para-phénylène, substitué ou non substitué, et que R est un groupe cyclohexyle, substitué ou non substitué, ou un groupe hydrocarboné bicyclique ou polycyclique saturé comportant 8—14 atomes de carbone, B ne représente pas un atome d'oxygène; et

(ii) si B est un atome d'oxygène, un groupe —$CO_2$— ou un groupe —NH—$CO_2$—, Ar soit un groupe para-phénylène ou para-pyridylène non substitué,

en faisant réagir un composé de formule générale

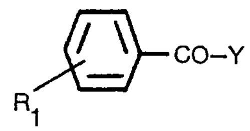

dans laquelle

$R_1$ a la signification indiquée ci-dessus, et

Y est un groupe amino ou un groupe isocyanato,

avec un composé de formule générale

R—B—Ar—Z

dans laquelle

R, B et Ar ont les significations indiquées ci-dessus, et

Z est un groupe isocyanato ou un groupe amino,

à condition que, si Y est un groupe amino, Z soit un groupe isocyanato, et que si Y est un groupe isocyanato, Z soit un groupe amino.

2. Procédé selon la revendication 1, caractérisé en ce que l'on prépare des composés benzoylurées de formule générale

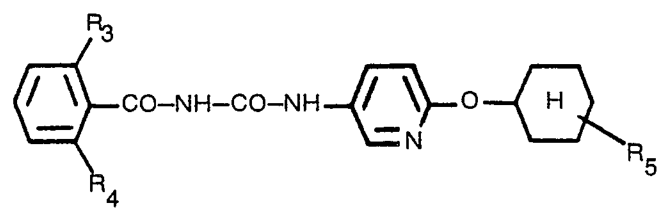

(II)

dans laquelle

$R_3$ est un atome d'hydrogène ou un atome d'halogène,

$R_4$ est un atome d'halogène, et

$R_5$ représente 1 à 3 groupes alkyle ou alcényle comportant respectivement 1—6 et 2—6 atomes de carbone, par un procédé selon la revendication 1.

3. Procédé selon la revendication 1, caractérisé en ce que l'on prépare des composés benzoylurée de formule générale

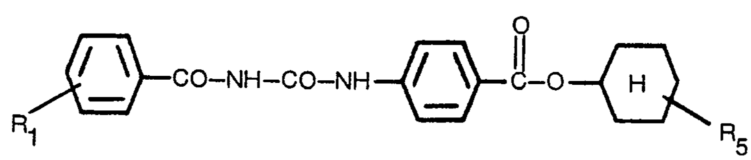

(III)

dans laquelle

$R_1$ a la signification indiquée dans la revendication 1, et

$R_5$ a la signification indiquée dans la revendication 2,

par un procédé selon la revendication 1.

4. Composition ayant une activité insecticide et acaricide, caractérisée en ce que, en plus d'un véhicule inerte liquide ou solide, la composition comprend un composé de formule générale I, dans laquelle $R_1$, Ar, B et R ont les significations indiquées à la revendication 1.

5. Composition selon la revendication 4, caractérisée en ce que le constituant actif est un composé de formule générale II, dans laquelle $R_3$, $R_4$ et $R_5$ ont les significations indiquées à la revendication 2.

6. Composition selon la revendication 4, caractérisée en ce que le constituant actif est un composé de formule générale III, dans laquelle $R_1$ a la signification indiquée à la revendication 1, et $R_5$ a la signification indiquée à la revendication 2.

7. Procédé de préparation d'une composition ayant une activité insecticide et acaricide, caractérisé en ce qu'un composé de formule générale I, dans laquelle $R_1$, Ar, B et R ont les significations indiquées à la revendication 1, est inclu dans un véhicule inerte liquide ou solide, éventuellement avec d'autres composés pesticides, des fumiers artificiels et/ou des substances auxiliaires.

8. Procédé de lutte contre les insectes et/ou les acariens, caractérisé en ce que l'on traite la zone infectée avec une composition selon l'une quelconque des revendications 4 à 6, en dose de 1 à 5000 g de substance active par hectare.